(19) Europäisches Patentamt
European Patent Office
Office européen des brevets

(11) EP 0 467 116 B2

(12) **NEUE EUROPÄISCHE PATENTSCHRIFT**

(45) Veröffentlichungstag und Bekanntmachung des Hinweises auf die Entscheidung über den Einspruch:
**05.12.2001 Patentblatt 2001/49**

(45) Hinweis auf die Patenterteilung:
**09.08.1995 Patentblatt 1995/32**

(21) Anmeldenummer: **91110528.6**

(22) Anmeldetag: **26.06.1991**

(51) Int Cl.[7]: **A61K 31/415**, A61K 7/48

(54) **Dermatologische Zusammensetzungen mit einem Gehalt an cis-Urocaninsäure**

Dermatological compositions containing cis-urocanic acid

Compositions dermatologiques contenant de l'acide cis-urocanique

(84) Benannte Vertragsstaaten:
**AT CH DE ES FR GB IT LI NL**

(30) Priorität: **29.06.1990 DE 4020739**

(43) Veröffentlichungstag der Anmeldung:
**22.01.1992 Patentblatt 1992/04**

(73) Patentinhaber: **GS DEVELOPMENT AB**
**213 75 Malmö (SE)**

(72) Erfinder:
- **Stäb, Franz, Dr.**
  **W-2171 Echem (DE)**
- **Sauermann, Gerhard, Dr.**
  **W-2351 Wiemersdorf (DE)**
- **Hoppe, Udo, Dr.**
  **W-2000 Hamburg 65 (DE)**
- **Engel, Walter**
  **W-2080 Pinneberg (DE)**

(74) Vertreter: **UEXKÜLL & STOLBERG**
**Patentanwälte**
**Beselerstrasse 4**
**22607 Hamburg (DE)**

(56) Entgegenhaltungen:
- **CHEMICAL ABSTRACTS, Band 92, Nr. 22, Juni 1980, Seite 287, Zusammenfassung Nr.185715c, Columbus, Ohio, US; S. OHNISHI et al.: "Study on urocanic acid in thehuman skin surface. II. Geometrical isomers and sunscreen effect",& J. SCCJ 1979, 13(2), 61-6**
- **ARCHIVES DE BIOCHEMIE ET COSMETOLOGIE, Band 11, Nr. 108, 1968, Seiten 21-28,Les Editions Varia, Paris, FR; J. MORELLE: "L'etat actuel de nos connaissancessur l'acide urocanique"**
- **Photochem.Photobiol., 1989, 50(2), pp.267-275 (Norval et al.)**

Bemerkungen:
Die Akte enthält technische Angaben, die nach dem Eingang der Anmeldung eingereicht wurden und die nicht in dieser Patentschrift enthalten sind.

EP 0 467 116 B2

Printed by Jouve, 75001 PARIS (FR)

## Beschreibung

Die vorliegende Erfindung betrifft die Verwendung einer wirksamen Menge an cis-Urocaninsäure und/oder deren Derivaten, insbesondere der Ester und der physiologisch verträglichen Salze, zur Herstellung dermatologischer Zubereitungen für die Behandlung und/oder die Prophylaxe von Psoriasis und Neurodermitis.

Dermatosen erzeugen bei den Betroffenen einen starken Leidensdruck. Die Anzahl entzündlicher oder allergischer Dermatosen steigt in den industrialisierten Ländern stetig. Untersuchungen belegen, daß berufsbedingte Hauterkrankungen dabei offensichtlich eine wesentliche Rolle spielen. Besonders Berufsdermatosen sind für die erkrankten Personen von existentieller Bedeutung, da sie in vielen Fällen einen Berufswechsel erzwingen. Zumindest aber erfordern sie verstärkte Vorsichtsmaßnahmen.

Produkte zur Behandlung dieser Erkrankungen sind an sich bekannt, insbesondere werden Antihistaminika oder Glucocorticoide eingesetzt. Zur Prophylaxe hingegen sind bislang keine geeigneten Präparate bekannt.

Die Zusammensetzungen des Standes der Technik, die mittlerweile in manchen handelsüblichen Formulierungen eingesetzt werden, haben jedoch einige Nachteile:
Antihistaminika verursachen bei vielen Personen Müdigkeit und Abgeschlagenheit. Permanente Anwendung von Glucocorticoiden (z.B. Cortison) ist wegen vieler unangenehmer Nebenwirkungen aus medizinischen Gründen meist nicht vertretbar. Ähnliches gilt auch für die meisten sogenannten NSAID (non steroidal anti-inflammatorial drugs).

Aufgabe der vorliegenden Erfindung war es somit, Wege zu finden, die Nachteile des Standes der Technik zu vermeiden. Insbesondere sollten Mittel zur Verfügung gestellt werden, die wirksam zur Behandlung und Prophylaxe von Psoriasis und Neurodermitis eingesetzt werden können, ohne daß die geschilderten Nebenwirkungen auftreten.

Erfindungsgemäß werden diese Aufgaben gelöst durch die Verwendung eines wirksamen Gehaltes an cis-Urocaninsäure und/oder deren Derivaten, insbesondere der Ester und der physiologisch verträglichen Salze, zur Herstellung dermatologischer Zubereitungen für die Behandlung und/oder Prophylaxe von Psoriasis und Neurodermitis.
cis-Urocaninsäure (auch cis-Urocansäure oder cis-4-Imidazolylacrylsäure genannt) ist durch folgende Strukturformel gekennzeichnet:

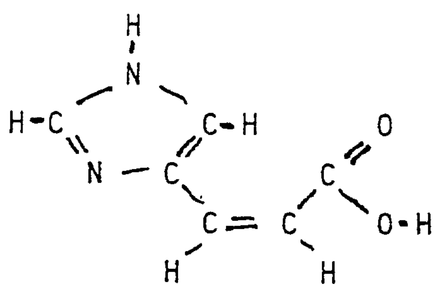

Sie hat die Summenformel $C_6H_6N_2O_2$ und die Molekularmasse 138,12. cis-Urocaninsäure entsteht beispielsweise durch UV-Bestrahlung des trans-Isomeren, welches in der menschlichen Haut und auch im Schweiß vorkommt. Die Verwendung des trans-Isomeren als Sonnenschutzmittel ist aus Chemical Abstracts, Band 82, Nr.22, Juni 1980, S. 287, Zusammenfassung Nr. 185715c bekannt.

Unter Derivaten der cis-Urocaninsäure werden in erster Linie verstanden ihre Ester und Salze, sowie auch die Produkte, die durch Substitution am Imidazolylrest entstehen.

Es hat sich in höchst überraschender Weise gezeigt, daß cis-Urocaninsäure antiphlogistisch wirkt, die Folgen allergischer Reaktionen mildert und in hohem Maße allergischen Reaktionen vorbeugt.

Aufgrund dieser antiphlogistischen und antiallergischen. Potenz sind die erfindungsgemäßen Zusammensetzungen wirksam gegen Psoriasis und Neurodermitis.

In den erfindungsgemäßen Zusammensetzungen liegt die cis-Urocaninsäure vorzugsweise in Konzentrationen von 0,00001 mg/ml - 60 mg/ml vor. Bevorzugt sind Zusammensetzungen mit Konzentrationen von 0,01 mg/ml - 2,0 mg/ml, insbesondere von 0,05 mg/ml - 1,0 mg/ml, jeweils bezogen auf die Gesamtmenge der Zusammensetzung.

Die erfindungsgemäßen Zusammensetzungen werden topisch, auf der Haut des Patienten angewandt. Bevorzugt sind mehrere Anwendungen am Tag in einer Zeit die zur Heilung des Patienten ausreicht, z.B. 14 Tage, und in einer genügend großen Menge.

Die erfindungsgemäßen Zusammensetzungen werden ebenfalls topisch, auf der Haut des Patienten angewandt, wenn Prophylaxe gewünscht ist. Bevorzugt sind mehrere Anwendungen am Tag in einer Zeit die zur Prophylaxe des Patienten ausreicht, z.B. 14 Tage, und in einer genügend großen Menge.

Erfindungsgemäße cis-Urocaninsäurehaltige Formulierungen können vorteilhaft gewählt werden aus allen

gängigen Anwendungsformen, z.B. Crèmes, Gele, Lotionen, Sprays, Milchen usw. Es hat sich als günstig erwiesen, wäßrige oder alkoholisch/wäßrige oder alkoholische oder acetonisch/wäßrige oder acetonische oder acetonisch/alkoholische Lösungen der cis-Urocaninsäure in die Formulierungen einzuarbeiten.

Es ist ferner von Vorteil, den Zusammensetzungen Hilfs- und/oder Zusatzstoffe einzuverleiben, die die Stabilität der cis-Urocaninsäure bzw. deren Derivate erhöhen oder die aus galenischer Sicht die Qualität der Zusammensetzungen verbessern oder verändern.

Hilfs- und Zusatzstoffe sind beispielsweise Verdicker, Füllstoffe, Farbstoffe, Emulgatoren, zusätzliche Wirkstoffe wie Lichtschutzmittel, Stabilisatoren, Antioxidantien, Penetrationsverstärker, Konservierungsmittel, Alkohol, Wasser, Salze, proteolytisch oder keratolytisch wirksame Substanzen usw.

Ganz besonders vorteilhaft ist, den erfindungsgemäßen Zusammensetzungen ungesättigte Fettsäuren zuzusetzen, da diese die Wirkung der cis-Urocaninsäure noch in überraschender Weise verstärken. Bevorzugt sind dabei gamma-Linolensäure, Eicosapentaensäure, Docosahexaensäure, Ölsäure sowie deren Derivate, vorteilhaft die entsprechenden Ester und Salze. Die ungesättigten Fettsäuren liegen vorzugsweise in Konzentrationen von 0,2 - 2,0 Gew.-% vor, bezogen auf das Gesamtgewicht der Zusammensetzung.

Es ist durchaus günstig, Gemische aus cis- und trans- Urocaninsäure zu verwenden. Dabei geht die erfindungsgemäße Wirkung zwar von der cis-Verbindung aus, dennoch kann der Anwender aus den Eigenschaften der trans-Verbindung Nutzen ziehen. Vorteilhaft ist es, das Racemat aus cis- und trans-Urocaninsäure zu verwenden (1 : 1).

Insbesondere ist es auch vorteilhaft, trans-Urocaninsäure Ultraviolettstrahlung auszusetzen, wobei ein Gemisch aus cis- und trans-Isomeren entsteht, und dieses Gemisch in die entsprechende Formulierung einzuarbeiten.

Ansonsten sind die üblichen Maßregeln zu beachten, die dem Fachmann bekannt sind.

Die folgenden Beispiele dienen dazu, die Erfindung zu beschreiben, ohne daß beabsichtigt ist, die

Erfindung auf diese Beispiele zu beschränken. cis-Urocaninsäure wird in den Beispielen abgekürzt als UCS.

Beispiel 1

| Wäßrige Zubereitung (Gesichtswasser) | |
|---|---|
| | Gew.-% |
| PEG-40-hydrogenated Castor Oil | 0,811 |
| Dipropylenglycol | 2,534 |
| PEG-8 | 1,521 |
| $Na_3EDTA$ | 0,253 |
| Polymer JR 125 | 0,025 |
| UCS | 0,750 |
| Wasser VES | ad 100,000 |

Beispiel 2

| Wäßrige Zusammensetzung | |
|---|---|
| | Gew.-% |
| Polyfettsäureester (Cetiol HE) | 16,000 |
| PPG-3-Myristylester (Witconol APM) | 1,000 |
| Propylenglycol | 3,000 |
| Glycerin | 40,000 |
| UCS | 0,500 |
| Wasser VES | ad 100,000 |

Beispiel 3

| Hydrogel (Polyacrylatgel) | |
|---|---|
| | Gew.-% |
| Acrylsäurepolymerisat (Carbopol 934) | 1,000 |
| Tris(hydromethylamino)methan (Tris) | 1,000 |
| Glycerin | 2,000 |
| Propylenglycol | 2,000 |
| UCS | 0,050 |
| Wasser VES | ad 100,000 |

Beispiel 4

| Hochwasserhaltige Zubereitung (sehr weich) | |
|---|---|
| | Gew.-% |
| Ceteareth (Cremophor A 25) | 0,100 |
| Cetearyl Alcohol (Lanette O) | 0,400 |
| Vaseline, DAB 9 | 12,500 |
| Mineralöl, DAB 9 | 11,000 |
| Ceteareth-6-stearylalkohol (Cremophor A6) | 6,000 |
| UCS | 0,020 |
| Wasser VES | ad 100,000 |

Beispiel 5

| Hochwasserhaltige Zubereitung (weich) | |
|---|---|
| | Gew.-% |
| Ceteareth-25 (Cremophor A25) | 1,500 |
| Cetearyl Alcohol (Lanette O) | 8,500 |
| UCS | 0,250 |
| Wasser VES | ad 100,000 |

Beispiel 6

| Hochwasserhaltige Zubereitung (weich) | |
|---|---|
| | Gew.-% |
| Ceteareth-25 (Cremophor A 25) | 2,000 |
| Cetearyl Alcohol (Lanette O) | 8,000 |
| Vaseline, DAB 9 | 10,000 |
| Mineralöl, DAB 9 | 10,000 |
| UCS | 0,100 |
| Wasser VES | ad 100,000 |

Beispiel 7

| Hochwasserhaltige Zubereitung (mittelfest) | |
|---|---|
| | Gew.-% |
| Ceteareth-25 | 3,000 |
| Cetearyl Alcohol (Lanette O) | 17,000 |
| UCS | 0,175 |
| Wasser VES | ad 100,000 |

Beispiel 8

| Dünnflüssige Lotion | |
|---|---|
| | Gew.-% |
| Ceteareth-25 (Cremophor A25) | 1,000 |
| Ceteareth-6-stearylalcohol (Cremophor A6) | 1,000 |
| Glycerin-mono-distereat (Tein normal) | 2,000 |
| Cetylalcohol | 1,000 |
| Isopropylmyristat | 1,450 |
| Glycerin | 1,000 |
| Polyvinylpyrrolidon | 0,500 |
| UCS | 1,125 |
| Wasser VES | ad 100,000 |

Beispiel 9

| Dickflüssige Lotion | |
|---|---|
| | Gew.-% |
| Ceteareth-25 (Cremophor A25) | 2,000 |
| Cetearyl Alcohol (Lanette O) | 3,000 |
| Mineralöl, DAB 9 | 5,000 |
| Propylenglycol | 3,000 |
| Polyvinylpyrrolidon | 0,500 |
| UCS | 0,300 |
| Wasser VES | ad 100,000 |

Beispiel 10

| W/O-Crème | |
|---|---|
| | Gew.-% |
| Glycerinsorbitanfettsäureester (Arlacel 481) | 6,000 |
| Mikokristallines Wachs (Lunacera M) | 1,000 |
| Neutrolöl | 3,000 |
| Paraffinöl | 19,000 |

(fortgesetzt)

| W/O-Crème | |
|---|---|
| | Gew.-% |
| Magnesiumstereat | 1,000 |
| Propylenglycol | 3,700 |
| Magnesiumsulfat ($MgSO_4$ *7 $H_2$ O) | 0,700 |
| UCS | 1,000 |
| Wasser VES | ad 100,000 |

Beispiel 11

| W/O-Emulsion | |
|---|---|
| | Gew.-% |
| Polyoxyethylen-Glycerin-Sorbitan-Fettsäureester (Arlacel 988) | 3,600 |
| Polyoxyethylen-Fettsäureester (Arlacel 989) | 1,400 |
| Cetearyl Alcohol (Lanette O) | 2,000 |
| Mineralöl, DAB 9 | 25,000 |
| Paraben-Mischung | nach Belieben |
| Magnesiumsulfat ($MgSO_4$ *7 $H_2$ O) | 0,700 |
| UCS | 1,250 |
| Wasser VES | ad 100,000 |

Beispiel 12

| W/O-Lotion | |
|---|---|
| | Gew.-% |
| Glycerinsorbitanfettsäureester (Arlacel 481) | 1,300 |
| Polyoxyethylen-Fettsäureester (Arlacel 989) | 3,700 |
| Neutralöl (Miglyol) | 6,000 |
| Paraffinöl, DAB 9 | 14,000 |
| Propylenglycol | 3,800 |
| Magnesiumsulfat ($MgSO_4$ *7 $H_2$ O) | 0,700 |
| UCS | 0,060 |
| Wasser VES | ad 100,000 |

Beispiel 13

| O/W-Emulsion | |
|---|---|
| | Gew.-% |
| PEG-100-Stearate (Arlacel 165) | 5,000 |
| Cetearyl Alcohol (Lanette O) | 3,000 |
| Mineralöl, DAB 9 | 25,000 |
| Paraben-Mischung | nach Belieben |
| UCS | 0,325 |

(fortgesetzt)

| O/W-Emulsion | |
|---|---|
| | Gew.-% |
| Wasser VES | ad 100,000 |

Beispiel 14

| O/W-Emulsion | |
|---|---|
| | Gew.-% |
| Polysorbate-60 (Tween 60) | 3,000 |
| Sorbitan Stearate (Arlacel 60) | 2,000 |
| Cetearyl Alcohol (Lanette O) | 3,000 |
| Mineralöl, DAB 9 | 25,000 |
| Paraben-Mischung | nach Belieben |
| UCS | 0,035 |
| Wasser VES | ad 100,000 |

Beispiel 15

| Kationenaktive Emulsion | |
|---|---|
| | Gew.-% |
| Distearyldimethylammoniumchlorid (Genamin DS AC) | 5,000 |
| Vaseline, DAB 9 | 5,000 |
| Isopropylpalmittat | 2,000 |
| Cetylalcohol | 1,000 |
| Siliconöl | 0,100 |
| Propylparaben | 0,100 |
| Methylparaben | 0,100 |
| Glycerin | 4,000 |
| UCS | 0,090 |
| Wasser VES | ad 100,000 |

Beispiel 16

| Ionische Emulsion | |
|---|---|
| | Gew.-% |
| Natrium Cetearylsulfat (Emulgade F) | 6,000 |
| Mineralöl, DAB 9 | 25,000 |
| Paraben-Mischung | nach Beliechen |
| UCS | 0,450 |
| Wasser VES | ad 100,000 |

Beispiel 17

| Ionische O/W-Emulsion | |
|---|---|
| | Gew.-% |
| Stearinsäure | 5,000 |
| Cetearyl Alcohol (Lanette O) | 3,000 |
| Mineralöl, DAB 9 | 25,000 |
| Paraben-Mischung | nach Belieben |
| Triethanolamin | 1,000 |
| UCS | 0,080 |
| Wasser VES | ad 100,000 |

Nachweis der durch cis-Urocaninsäure bewirkten Suppression der durch DNFB (Dinitrofluorbenzol) induzierten Kontaktallergie (vergleichsversuche bezuglich Kontaktallergie).

Material und Methoden

Versuch (1)

Testtiere: Als Testtiere wurden zehn naive, syngene weibliche Mäuse (Alter: 12 - 14 Wochen) je Gruppe in den Experimenten gewählt.

Urocaninsäure: trans-Urocaninsäure wurde von Sigma (München, BRD) geliefert. Für die Isomerisierung wurde die trans-Urocaninsäurelösung (1 % in doppelt destilliertem Wasser, mit NaOH auf pH 6,9 gepuffert) mit einer Osram Vitalux Lampe bestrahlt, wobei eine 50-%-ige Umwandlung des trans- zum cis-Isomeren erfolgte, kontrolliert durch HPLC-Analyse.

Die Lösung des cis/trans-Racemates wurde durch einen 0,1 µm Millipore Filter passiert und in die Wasserphase eine O/W-Crème (1 mg Urocaninsäure / ml Crème). Die O/W-Crème ohne Urocaninsäure (Placebo) diente als Kontrolle (siehe Tabelle 3).

Induktion und Nachweis der Kontaktallergie auf DNFB: (Vergleichsversuche bezugliche Kontaktallergie)

DNFB wurde von Sigma (München, BRD) geliefert und als Allergen benutzt, um eine Kontaktallergie (Delayed Hypersensitivity Reaction, DH) hervorzurufen. Vor der Sensibilisierung mit DNFB wurden 10 Mäuse je Gruppe während des Verlaufs von 3 Tagen täglich mit 100 µl Urocaninsäurecrème (Gruppe A) bzw. Placebo (Gruppe B) auf einer rasierten Hautfläche (6 $cm^2$) der Bauchregion behandelt.

5 Stunden nach der letzten topischen Applikation der Crèmes wurden die Mäuse beider Gruppen durch epikutane Behandlung der rasierten Hautflächen mit je 30 µl einer 0,6-%-igen DNFB-Lösung in Aceton/Olivenöl (4 : 1) sensitiviert.

8 Tage nach der Sensibilisierung wurden die Mäuse der Gruppe A mit 10 µl der Urocaninsäurecrème (Datengruppe A II) auf der Oberfläche eines Ohres bzw. 10 µl der Placebocrème (Datengruppe A I) auf der Oberfläche des anderen Ohres eingerieben. Gleichzeitig wurden die Mäuse der Gruppe B mit 10 µl der Urocaninsäurecrème (Datengruppe B II) auf der Oberfläche eines Ohres bzw. 10 µl der Placebocrème (Datengruppe B I) auf der Oberfläche des anderen Ohres eingerieben. Die Verteilung der behandelten Ohren (links oder rechts) wurde in beiden Tiergruppen durch Zufall entschieden.

Einen Tag darauf wurde die Dicke beider Ohren aller Gruppen mittels eines Mikrometers gemessen, um die Grundwerte zu ermitteln.

Danach wurden alle Mäuse auf der Oberfläche beider Ohren mit je 20 µl DNFB (0,5 %) behandelt. 24 Stunden später wurde die Dicke der Ohren erneut gemessen.

Die DH-Antwort wurde als der durchschnittliche Zuwachs der Ohrendicke je Maus berechnet (netto-Ohrenschwellung). Der Prozentsatz der DH-Suppression für die Ohren mit bzw. ohne Wirkstoffbehandlung wurde gemäß der Formel

$$\text{DH-Suppr.} = 100 - \frac{100 * \text{netto-Ohrenschwellung (Gruppe A)}}{\text{netto-Ohrenschwellung (Gruppe B)}} \ (\%)$$

Die statistische Signifikanz der Ergebnisse wurde durch den Wilcoxon U-Test errechnet.

Ergebnisse (s. Tabelle 1):

Die arithmetischen Mittelwerte der Ohrendicke in beiden Mäusegruppen vor der DNFB-Behandlung waren statistisch nicht unterschiedlich. Jedoch 24 Stunden nach der DNFB-Behandlung war die Ohrendicke der Datengruppe B I (s. Tabelle 1) im Vergleich zur Datengruppe A I signifikant angewachsen.
In Datengruppe A II war die DH-Antwort im Vergleich zu Datengruppe B I hochsignifikant supprimiert (58,3 % Suppression) und im Vergleich zu den Datengruppen A I und B II signifikant supprimiert.
Diese Daten beweisen, daß entweder prophylaktische Behandlung mit cis-Urocaninsäure enthaltenden Formulierungen oder, falls die Sensibilisierung bereits eingetreten die Behandlung mit cis-Urocaninsäure enthaltenden Formulierungen, Schutz vor DH-Reaktionen bietet.

Tabelle 1:

| Effekt von O/W-Crèmes mit und ohne Urocaninsäure auf die DH-Antwort gegen DNFB | | | |
|---|---|---|---|
| Datengruppe | von Tag 1-3 behandelt mit (Abdomen) | am Tage 11 behandelt mit (Ohr) | Ohrschwellung am Tage 13 |
| A I | Wirkstoff | Placebo | 7,96 (0,90) |
| A II | Wirkstoff | Wirkstoff | 4,95 (0,98) |
| B I | Placebo | Placebo | 11,87 (1,07) |
| B II | Placebo | Wirkstoff | 7,33 (0,57) |
| Wirkstoff = Crème mit Urocaninsäure<br>Placebo = Crème ohne Urocaninsäure | | | |

Die Werte für die Ohrschwellung (Dickenzuwachs) wurden gemessen in mm * $10^{-2}$. Die Werte in Klammern bedeuten die Standardabweichung des Mittelwertes.
Die Verhältnisse der Gruppen zueinander (Suppression der Ohrschwellung in %) betragen:

AI:BI = 32,1 %
A II : A I = 37,9 %
A II : B I = 58,3 %
A II : B II = 32,5 %
B II : A I = 8,0 %
B II : B I = 38,3 %

Versuch (2)

Testtiere: Wie in Versuch (1)

Urocaninsäure: Wie in Versuch (1)

Die Lösung des cis/trans-Racemates wurde durch einen 0,1 µm Millipore Filter passiert und in die Wasserphase eine O/W-Crème (1 mg Urocaninsäure / ml Crème) eingearbeitet. Die Crème enthielt zusätzlich 0,75 Gew.-% gamma-Linolensäure.
Die Crème ohne Urocaninsäure, aber mit gamma-Linolensäure (Placebo) diente als Kontrolle (s. Tabelle 2).

Induktion und Nachweis der Kontaktallergie auf DNFB: (Vergleichsversuche bezugliche Kontaktallergie)

Vor der Sensibilisierung mit DNFB wurden 10 Mäuse je Gruppe wahrend des Verlaufs von 3 Tagen täglich mit 100 µl Urocaninsäurecrème (Gruppe C) bzw. Placebo (Gruppe D) auf einer rasierten Hautfläche (6 $cm^2$) der Bauchregion behandelt.
5 Stunden nach der letzten topischen Applikation der Crèmes wurden die Mäuse beider Gruppen durch epikutane Behandlung der rasierten Hautflächen mit je 30 µl einer 0,6-%-igen DNFB-Lösung in Aceton/Olivenöl (4 : 1) sensitiviert.
8 Tage nach der Sensitivierung wurden die Mäuse der Gruppe C mit 10 µl der Urocaninsäurecrème (Datengruppe C II) auf der Oberfläche eines Ohres bzw. 10 µl der Placebocrème (Datengruppe C I) auf der Oberfläche des

anderen Ohres eingerieben. Gleichzeitig wurden die Mäuse der Gruppe D mit 10 µl der Urocaninsäurecrème (Datengruppe D II) auf der Oberfläche eines Ohres bzw. 10 µl der Placebocrème (Datengruppe D I) auf der Oberfläche des anderen Ohres eingerieben. Die Verteilung der behandelten Ohren (links oder rechts) wurde in beiden Tiergruppen durch Zufall entschieden.

Einen Tag darauf wurde die Dicke beider Ohren aller Gruppen mittels eines Mikrometers gemessen, um die Grundwerte zu ermitteln.

Danach wurden alle Mäuse auf der Oberfläche beider Ohren mit je 20 µl DNFB (0,5 %) behandelt. 24 Stunden später wurde die Dicke der Ohren erneut gemessen.

Die DH-Antwort wurde als der durchschnittliche Zuwachs der Ohrendicke je Maus berechnet (netto-Ohrenschwellung). Der Prozentsatz der DH-Suppression für die Ohren mit bzw. ohne Wirkstoffbehandlung wurde gemäß der Formel

$$\text{DH-Suppr.} = 100 - \frac{100 * \text{netto-Ohrenschwellung (Gruppe C)}}{\text{netto-Ohrenschwellung (Gruppe D)}}\ (\%)$$

Die statistische Signifikanz der Ergebnisse wurde durch den Wilcoxon U-Test errechnet.

<u>Ergebnisse (s. Tabelle 2):</u>

Die arithmetischen Mittelwerte der Ohrendicke in beiden Mäusegruppen vor der DNFB-Behandlung waren statistisch nicht unterschiedlich. Jedoch 24 Stunden nach der DNFB-Behandlung war die Ohrendicke der Datengruppe D I (s. Tabelle 2) im Vergleich zu Datengruppe C I signifikant angewachsen.

In Datengruppe C II war die DH-Antwort im Vergleich zu Datengruppe D I hochsignifikant supprimiert (65,6 % Suppression) und im Vergleich zu den Datengruppen C I und D II signifikant supprimiert.

Diese Daten beweisen, daß entweder prophylaktische Behandlung mit cis-Urocaninsäure und gamma-Linolensäure enthaltenden Formulierungen oder, falls die Sensitivierung bereits eingetreten ist, die Behandlung mit cis-Urocaninsäure und gamma-Linolensäure enthaltenden Formulierungen, Schutz vor DH-Reaktionen bei Allergenkontakt bietet.

Tabelle 2:

| Effekt von O/W-Crèmes mit und ohne Urocaninsäure auf die DH-Antwort gegen DNFB | | | |
|---|---|---|---|
| Datengruppe | von Tag 1-3 behandelt mit (Abdomen) | am Tage 11 behandelt mit (Ohr) | Ohrschwellung am Tage 13 |
| C I | Wirkstoff | Placebo | 7,74 (0,87) |
| C II | Wirkstoff | Wirkstoff | 4,02 (1,02 |
| D I | Placebo | Placebo | 11,70 (1,01) |
| D II | Placebo | Wirkstoff | 6,99 (0,70) |
| Wirkstoff = Crème mit Urocaninsäure und gamma-Linolensäure<br>Placebo = Crème ohne Urocaninsäure, mit gamma-Linolensäure | | | |

Die Werte für die Ohrschwellung (Dickenzuwachs) wurden gemessen in mm $*10^{-2}$. Die Werte in Klammern bedeuten die Standardabweichung des Mittelwertes.

Die Verhältnisse der Gruppen zueinander (Suppression der Ohrschwellung in %) betragen:

CI:DI = 33,8 %
CII:CI = 48,1 %
CII:DI = 65,6 %
CII:DII = 42,5 %
DII:CI = 9,7 %
DII:DI = 40,3 %

Versuch (3)

Versuch auf antiphlogistische Wirkung:

Nach UV-Bestrahlung (Sol 3, Firma Hönle) wurde die bestrahlte Haut (Fläche der Bestrahlungsfenster 1,0 *

1,2 $cm^2$, Rücken der Testpersonen) mit den Testpräparaten eingecremt.

(a) Testpräparat A wurde bei 14 Probanden (Alter : 31,2 Jahre (Mittelwert), Standardabweichung 7,7 Jahre) unmittelbar nach UV-Bestrahlung und ein weiteres Mal nach 6 Stunden aufgetragen. Die Crèmes wurden abgewogen, so daß die applizierte Menge 2 $mg/cm^2$ entsprach.
(b) Testpräparat B wurde bei 14 Probanden unmittelbar nach UV-Bestrahlung und ein weiteres Mal nach 6 Stunden aufgetragen. Die Crèmes wurden abgewogen, so daß die applizierte Menge 2 $mg/cm^2$ entsprach.
(c) Testpräparat C wurde bei 14 Probanden unmittelbar nach UV-Bestrahlung und ein weiteres Mal nach 6 Stunden aufgetragen. Die Crèmes wurden abgewogen, so daß die applizierte Menge 2 $mg/cm^2$ entsprach.
(d) Testpräparat D wurde bei 14 Probanden unmittelbar nach UV-Bestrahlung und ein weiteres Mal nach 6 Stunden aufgetragen. Die Crèmes wurden abgewogen, so daß die applizierte Menge 2 $mg/cm^2$ entsprach.

Egebnisse:

Die entstehenden Erytheme wurden 24 Stunden nach Bestrahlung visuell beurteilt:

(a) 12 Personen: schwaches Erythem, 2 Personen: deutliches Erythem
(b) 13 Personen: deutliches Erythem, 1 Person: schwaches Erythem
(c) 13 Personen: kein Erythem, 1 Person: schwaches Erythem
(d) 10 Personen: deutliches Erythem, 3 Personen leichtes Erythem, 1 Person: schweres Erythem

Versuch (4) (Vergleichsversuche)

Auf die volaren Unterarme einer Versuchsperson mit Nickelallergie wurde 3 Tage lang, einmal täglich, eine O/W-Crème der Zusammensetzung E (linker Arm) und eine O/W-Crème der Zusammensetzung F (rechter Arm, Placebo) aufgetragen. Unmittelbar nach dem letzten Auftragen wurden beide Unterarme mit 20 µl einer Zubereitung aus 0,1 Gew.-% $NiSO_4$ in Vaseline behandelt. Die Reaktionen wurden 72 Stunden nach Applikation des Allergens visuell beurteilt. Wo der mit Placebo behandelte Unterarm eine deutliche allergische Reaktion zeigte, war am mit Wirkstoff behandelten Arm nur eine sehr schwache Rötung sichtbar.

Versuch (5)

Eine an Psoriasis leidende Versuchsperson wurde über einen Zeitraum von sechs Wochen mit

(5.1) einer Zusammensetzung gemäß Crème A in der Gegend um den linken Ellbogen
(5.2) einer Zusammensetzung gemäß Crème B in der Gegend um den rechten Ellbogen behandelt.

Beide Ellbogen zeigten vor dem Versuch Psoriasissymptome, nämlich Plaqueformen).

Ergebnis:

Nach sechs Wochen waren die Psoriasissymptome am linken Arm zurückgegangen, am rechten Arm waren sie unverändert geblieben.

Versuch (6)

Eine an schwerer Psoriasis leidende Versuchsperson wurde über einen Zeitraum von sechs Wochen mit

(6.1) einer Zusammensetzung gemäß Crème C in der Gegend um den linken Ellbogen
(6.2) einer Zusammensetzung gemäß Crème D in der Gegend um den rechten Ellbogen behandelt.

Beide Ellbogen zeigten vor dem Versuch schwere Psoriasissymptome, nämlich Plaqueformen.

Ergebnis:

Nach sechs Wochen waren die Psoriasissymptome am linken Arm zurückgegangen, am rechten Arm waren sie unverändert geblieben.

Tabelle 3

| Zusammensetzungen gemäß den Versuchen (1) - (6) | |
|---|---|
| Die wirkstoffhaltige Crème A hatte die Zusammensetzung | |
| | Gew.-% |
| Ceteareth-20 | 3,00 |
| Cetylstearylalcohol | 8,00 |
| Vaseline | 10,00 |
| Mineralöl | 10,00 |
| UCS | 1,00 |
| Wasser VES | 68,00 |
| Der Placebo B hatte die Zusammensetzung | |
| | Gew.-% |
| Ceteareth-20 | 3,00 |
| Cetylstearylalcohol | 8,00 |
| Vaseline | 10,00 |
| Mineralöl | 10,00 |
| Wasser VES | 69,00 |
| Die wirkstoffhaltige Crème C hatte die Zusammensetzung | |
| | Gew.-% |
| Ceteareth-20 | 3,00 |
| Cetylstearylalcohol | 8,00 |
| Vaseline | 10,00 |
| Mineralöl | 10,00 |
| UCS | 1,00 |
| gamma-Linolensäure | 0,75 |
| Wasser VES | 67,25 |
| Der Placebo D hatte die Zusammensetzung | |
| | Gew.-% |
| Ceteareth-20 | 3,00 |
| Cetylstearylalcohol | 8,00 |
| Vaseline | 10,00 |
| Mineralöl | 10,00 |
| gamma-Linolensäure | 0,75 |
| Wasser VES | 68,25 |
| Die wirkstoffhaltige Crème E hatte die Zusammensetzung | |
| | Gew.-% |
| Ceteareth-20 | 3,00 |
| Cetylstearylalcohol | 8,00 |
| Vaseline | 10,00 |
| Mineralöl | 10,00 |
| UCS | 2,00 |
| Wasser VES | 67,00 |

Tabelle 3 (fortgesetzt)

| Der Placebo F hatte die Zusammensetzung | |
|---|---|
| | Gew.-% |
| Ceteareth-20 | 3,00 |
| Cetylstearylalcohol | 8,00 |
| Vaseline | 10,00 |
| Mineralöl | 10,00 |
| Wasser VES | 69,00 |

**Patentansprüche**

1. Verwendung einer wirksamen Menge an cis-Urocaninsäure und/oder deren Derivaten, insbesondere der Ester und der physiologisch verträglichen Salze, zur Herstellung dermatologischer Zubereitungen für die Behandlung und/oder die Prophylaxe von Psoriasis und Neurodermitis.

2. Verwendung nach Anspruch 1, bei der die dermatologischen Zubereitungen einen Gehalt an 0,00001 mg/ml bis 60 mg/ml cis-Urocaninsäure aufweisen.

3. Verwendung nach Anspruch 1 oder 2, **dadurch gekennzeichnet, daß** die dermatologischen Zubereitungen einen zusätzlichen Gehalt an ungesättigten Fettsäuren und/oder deren Derivaten aufweisen.

4. Verwendung nach Anspruch 3, **dadurch gekennzeichnet, daß** die ungesättigten Fettsäuren aus der Gruppe bestehend aus gamma-Linolensäure, Eicosapentaensäure, Docosahexaensäure, Ölsäure, sowie deren Derivate, vorteilhaft die entsprechenden Ester und Salze, ausgewählt sind.

5. Verwendung nach einem der Ansprüche 1 bis 4, **dadurch gekennzeichnet, daß** die Zubereitungen ein Gemisch aus cis- und trans-Urocaninsäure enthalten, insbesondere cis- und trans-Urocaninsäure im Verhältnis 1 : 1.

**Claims**

1. Use of an effective amount of cis-urocaninic acid and/or its derivatives, in particular of the esters and of the physiologically tolerable salts, for the production of dermatological preparations for the treatment and/or prophylaxis of psoriasis and neurodermatitis.

2. Use according to claim 1, in which the dermatological preparations have a content of 0.00001 mg/ml to 60 mg/ml of cis-urocaninic acid.

3. Use according to claim 1 or 2, **characterized in that** the dermatological preparations have an additional content of unsaturated fatty acids and/or their derivatives.

4. Use according to claim 3, **characterized in that** the unsaturated fatty acids are selected from the group consisting of gamma-linolenic acid, eicosapentaenoic acid, docosahexaenoic acid, oleic acid, and their derivatives, advantageously the corresponding esters and salts.

5. Use according to one of claims 1 to 4, **characterized in that** the preparations contain a mixture of cis- and trans-urocaninic acid, in particular cis- and trans-urocaninic acid in a ratio of 1:1.

**Revendications**

1. Utilisation d'une quantité efficace en acide cis-urocanique et/or ses dérivés, en particulier les esters et les sels physiologiquement acceptables, pour la préparation de compositions dermatologiques destinées au traitement et/ou à la prophylaxie de psoriasis et neurodermitis.

**2.** Utilisation selon la revendication 1, dans laquelle les compositions dermatologiques ont une teneur en acide cis-urocanique allant de 0,00001 à 60 mg/ml.

**3.** Utilisation selon la revendication 1 ou 2, **caractérisée en ce que** les compositions dermatologiques ont une teneur supplémentaire en acides gras insaturés et/or leurs dérivés.

**4.** Utilisation selon la revendication 3, **caractérisée en ce que** les acides gras insaturés sont choisis dans le groupe constitué par l'acide γ-linolénique, l'acide eicosapentaénoïque, l'acide docosahexaénoïque, l'acide oléique et leurs dérivés, avantageusement les esters et les sels correspondants.

**5.** Utilisation selon l'une des revendications 1 à 4, **caractérisée en ce que** les compositions contiennent un mélange d'acide cis-urocanique et d'acide trans-urocanique, en particulier l'acide cis-urocanique et l'acide trans-urocanique au prorata de 1 à 1.